## Europäisches Patentamt
## European Patent Office
## Office européen des brevets

(11) Veröffentlichungsnummer : **0 377 809 B1**

(12) **EUROPÄISCHE PATENTSCHRIFT**

(45) Veröffentlichungstag der Patentschrift :
**08.07.92 Patentblatt 92/28**

(51) Int. Cl.⁵ : **C07C 49/597**, C07C 45/59

(21) Anmeldenummer : **89121272.2**

(22) Anmeldetag : **17.11.89**

(54) **Verfahren zur Herstellung von 3-Methyl-2-pentyl-cyclopent-2-en-1-on.**

(30) Priorität : **13.01.89 DE 3900815**

(43) Veröffentlichungstag der Anmeldung :
**18.07.90 Patentblatt 90/29**

(45) Bekanntmachung des Hinweises auf die
Patenterteilung :
**08.07.92 Patentblatt 92/28**

(84) Benannte Vertragsstaaten :
**AT BE CH DE ES FR GB IT LI NL SE**

(56) Entgegenhaltungen :
**DE-B- 1 107 222
DE-C- 693 863
PATENT ABSTRACTS OF JAPAN, Band 7, Nr.
285 (C-201)[1430], 20. Dezember 1983; & JP-
A-58 162 548**

(73) Patentinhaber : **HÜLS
AKTIENGESELLSCHAFT
Patentabteilung / PB 15 - Postfach 13 20
W-4370 Marl 1 (DE)**

(72) Erfinder : **Claus, Harald, Dr.
Markenkamp 41
W-4358 Haltern (DE)**

EP 0 377 809 B1

**Beschreibung**

Die saure Cyclodehydratisierung der leicht zugänglichen alkylsubstituierten gamma-Lactone zu alkylierten Cyclopent-2-enonen ist seit langem bekannt. So wurde bereits gemäß FR-PS 765 515 gamma-Undecalacton mittels konzentrierter Schwefelsäure zu 2-Hexylcyclopent-2-enon und gemäß DRP 639 455 gamma-Methyl-gamma-decalacton (5-Hexyl-5-methyl-dihydro-2(3H)-furanon) bei 300 °C an Silicagel zu 3-Methyl-2-pentyl-cyclopent-2-en-1-on ("Dihydrojasmon") cyclodehydratisiert. "Dihydrojasmon" ist ein bekannter Riechstoff.

Das erste Verfahren mit hohen Ausbeuten wurde von C. Rai und S. Dev veröffentlicht (J. Indian Chem. Soc. 34, 178 (1957). Die Autoren verwendeten Polyphosphorsäure in Form eines festen Gemisches aus Phosphorpentoxid und 85 %iger Phosphorsäure, von dem sie jedoch mehr als 24 Moläquivalente zum vollständigen Umsatz der eingesetzten Lactone bei 97 °C benötigten. Anschließend wurde das Produkt aus der festen Polyphosphorsäure extrahiert. Ein solches Verfahren ist aber in der Technik schon aufgrund der Korrosion heißer Phosphorsäureschmelzen und der Entsorgung der mit organischen Verbindungen verunreinigten Phosphorsäureabwässer nicht durchführbar.

Das von P. E. Eaton et al. (J. Org. Chem. 38, 4071 (1973) entwickelte flüssige Reagenz aus 10 Gewichtsteilen Methansulfonsäure und 1 Gewichtsteil Phosphorpentoxid wirft ebenfalls erhebliche technische Probleme auf. Erstens benötigte man zum Umsatz von gamma-Methyl-gamma-decalacton zum Dihydrojasmon die 80fache Gewichtsmenge dieses Reagenzes, zweitens ist das Gemisch so aggressiv, daß es nur bei Raumtemperatur unter Inkaufnahme langer Reaktionszeiten (33 h) eingesetzt wurde, und drittens erzeugt die Extraktion der wasserlöslichen Methansulfonsäure mit organischen Verbindungen erheblich belastete Abwässer.

Gemäß DE-OS 24 39 742 erfolgt u. a. die Cyclodehydratisierung von gamma-Lactonen an festen Säuren bei hohen Temperaturen. So wurde gamma-Methyl-gamma-decalacton durch Flashpyrolyse an 350 °C heißem Borphosphat zu einem Gemisch umgesetzt, das 84 GC-% an Dihydrojasmon und noch 4 GC-% an Edukt enthielt. Zwar wurde hier nur 10 Gew.-%, bezogen auf Lacton, an Katalysator verbraucht, der Austausch und die Entsorgung des polyesterverklebten, festen Katalysators ist in der Praxis aber viel zu kostenintensiv.

Es war daher Aufgabe der Erfindung, nach einem Verfahren zur Cyclodehydratisierung von gamma-Methyl-gamma-decalacton (5-Hexyl-5-methyldihydro-2(3H)-furanon) zu 3-Methyl-2-pentyl-cyclopent-2-en-1-on zu entwickeln, welches die vorstehend genannten gravierenden Nachteile vermeidet.

Gegenstand der Erfindung ist daher ein Verfahren zur Herstellung von 3-Methyl-2-pentyl-cyclopent-2-en-1-on durch katalytische Cyclodehydratisierung von 5-Hexyl-5-methyldihydro-2(3H)-furanon (gamma-Methyl-gamma-decalacton) in Gegenwart von Phosphorsäure bei erhöhter Temperatur, welches dadurch gekennzeichnet ist, daß als Katalysator ein Gemisch von 5 bis 20 Gew.-% an Phosphorsäure und einem hochsiedenden Mineralöl eingesetzt, die Reaktion bei 145 bis 175 °C unter Entfernung des entstehenden Produktes sowie des Reaktionswassers unter Anlegen eines Unterdruckes durchgeführt wird.

Geeignet sind alle Mineralöle, deren Siedepunkt oberhalb der Siedepunkte von Produkt und Ausgangsstoff liegen, z. B. Weißöle, die aus einem Gemisch gesättigter Alkane und Cycloalkane mit Siedebereichen von 350 bis 410 °C und 385 bis 485 °C bestehen.

Die Größe des angewendeten Unterdrucks ergibt sich aus dem Siedepunkt des Produktes sowie dem Druckverlust der verwendeten Destillationskolonne.

Mit dem erfindungsgemäßen Katalysator ist es möglich, 3-Methyl-2-pentyl-cyclopent-2-en-1-on in einem kontinuierlichen Verfahren in guten Ausbeuten zu gewinnen. Hierzu tropft man das entsprechende Lacton in den 145 bis 175 °C, insbesondere 150 bis 170 °C heißen Katalysator und destilliert das entstehende Produkt bei ausreichendem Unterdruck über eine Destillationskolonne genügender Trennleistung ab, wobei das freiwerdende Reaktionswasser in der Regel gasförmig entfernt wird. Selbstverständlich kann das Verfahren auch diskontinuierlich geführt werden. Die Phosphorsäure kann als 100 %ige Säure, aber auch in Form von wäßriger Lösung eingesetzt werden. Bezogen auf 1 Mol Phosphorsäure im Katalysator ist die Aktivität des Katalysators nach Umsatz von 10 Mol des Lactons unverändert hoch. Nach Umsatz von 20 Mol des Lactons beträgt die Aktivität noch 80 bis 90 % des Anfangswertes.

Zusammenfassend zeigt das erfindungsgemäße Verfahren folgende Vorteile:

1. Das Katalysatorgemisch aus Phosphorsäure und hochsiedendem Mineralöl ist flüssig und leicht handhabbar.

2. Es treten unter den angegebenen Bedingungen keine Korrosionsprobleme an Nickellegierungen, wie Hastalloy B oder C, auf.

3. Es werden nur katalytische Mengen an Phosphorsäure verbraucht.

4. Das Katalysatorgemisch kann problemlos aufgearbeitet werden, indem man geringe Mengen an Kohlenwasserstofflösungsmittel und Wasser zusetzt und die untenliegende klare Phosphorsäurephase abtrennt. Diese regenerierte Phosphorsäure ist direkt wiedereinsetzbar.

5. Es fallen keine mit organischen Verbindungen belasteten Abwässer an!

6. Milde Reaktionsbedingungen und geringe Verweilzeiten des gebildeten Produkts in der Reaktionszone erlauben hohe Ausbeuten an dem nahezu isomerreinen Cyclopent-2-enon.

Die Erfindung wird durch die nachstehenden Beispiele näher erläutert:

Beispiel 1

Ein 2 l-Dreihalskolben mit Innenthermometer, Magnetrührer, Tropftrichter, beheizbarer Destillationskolonne (Höhe = 50 cm, Innendurchmesser = 30 mm, V$_4$A-Multifilpackung) mit Rücklaufteiler, Kühler und Wechselvorlage wird mit 900 g hochsiedendem Weißöl, 100 g (1 mol) 98 %iger Phosphorsäure und 184,3 g (1 mol) 5-Hexyl-5-methyldihydro-2(3H)-furanon beschickt. Man heizt den Kolonnenmantel auf 65 °C, legt einen Kopfdruck von 3 hPa an und erhitzt den Kolbeninhalt auf 160 °C Sumpftemperatur.

Bei geschlossenem Rücklaufteiler läßt man über ca. 3 h die entstehenden Leichtsieder sich in der Kolonne ansammeln. Danach öffnet man den Rücklaufteiler zeittaktgesteuert im Verhältnis 10 : 1 und destilliert die entstehenden Produkte kontinuierlich ab (Kopftemperatur ca. 90 °C). Das entstehende Reaktionswasser geht bei diesem Innendruck gasförmig über und wird in einer Kühlfalle ausgefroren.

Die jeweils nach 1 h abdestillierte Produktmenge wurde durch Zugabe der 1,1fachen Menge an 5-Hexyl-5-methyldihydro-2(3H)-furanon ersetzt, die über den Tropftrichter in den Reaktionssumpf gegeben werden.

Will man die kontinuierliche Reaktion beenden, so stoppt man die weitere Zugabe von 5-Hexyl-5-methyl-dihydro-2(3H)-furanon und destilliert so lange weiter ab, bis der nachgebildete Produktstrom versiegt. Anschließend läßt man den Sumpf auskühlen, belüftet die Apparatur und fügt dem Sumpf mindestens 10 Gew.-% Wasser und zur besseren Phasentrennung 10 bis 20 Gew.-% eines Kohlenwasserstofflösungsmittels, wie zum Beispiel Cyclohexan, zu. Das Gemisch wird bei ca. 60 bis 80 °C gut durchgerührt und die untenliegende wäßrige Phosphorsäurephase abgetrennt, die nach einer Gehaltsbestimmung direkt wieder in einen neuen Versuch einsetzbar ist. Die im Reaktor verbleibende organische Phase wird fraktioniert, um das eingesetzte Lösungsmittel, Produkt- und Eduktreste zurückzugewinnen. Die verbleibende Weißölphase kann 2 bis 3mal wieder in einen neuen Versuch eingesetzt werden, am Ende ihres Lebenscyclus kann sie problemlos verbrannt werden.

Nach dieser Verfahrensweise kann man mit 1 mol Phosphorsäure bei konstanter Abdestillationsrate 10 mol 5-Hexyl-5-methyl-2(3H)-furanon zu 3-Methyl-2-pentylcyclopent-2-en-1-on umsetzen. Erst nach einem Umsatz von 20 mol des Lactons reduziert sich die Produktbildungsrate um 10 bis 20 %.

Das abdestillierte Produktgemisch enthält neben 90 bis 95 % GC-Flächen-% an 3-Methyl-2-pentylcyclopent-2-en-1-on weitere leichtersiedende Abbauprodukte und 1 bis 2 mol-% (lt. NMR-Integration) an dem Produktisomeren 2-Pentylcyclohex-2-en-1-on. Diese leichtsiedenden Nebenkomponenten trennt man durch Andestillieren des Produktgemisches in der oben beschriebenen Apparatur ab.

```
Ausbeute nach Umsatz
von 10 mol Lacton    :  1 498,1 g (9,01 mol) ≙ 90 % Ausbeute an
                        3-Methyl-2-pentylcyclopent-2-en-1-on

Ausbeute nach Umsatz
von 20 mol Lacton    :  2 893,8 g (17,04 mol) ≙ 87 % Ausbeute an
                        3-Methyl-2-pentylcyclopent-2-en-1-on

typische Abtragungs-
raten an Hastalloy-
C₄-Blechen           :  0,01 - 0,02 mm/Jahr
```

Beispiel 2

Man verfährt nach Beispiel 1, setzt aber 5-Hexyl-5-methyldihydro-2(3H)-furanon nicht in Abhängigkeit der momentanen Produktabdestillationsrate zu, sondern mit einer konstanten Menge pro Zeiteinheit (an der beschriebenen Apparatur mit 30 ml/h ≈ 28 g/h). Dieses Verfahren gewährt einen gleichmäßigeren Siedeverlauf und eine bessere Kolonnenauslastung. Weiterhin wird statt 98 %iger nun 115,3 g (1 mol) 85 %iger Phosphorsäure eingesetzt.

```
Ausbeute nach Umsatz
von 10 mol Lacton      :  1 546,0 g (9,30 mol) ≙ 93 % Ausbeute an
                          3-Methyl-2-pentylcyclopent-2-en-1-on
```

Beispiel 3

Man führt den Versuch nach Beispiel 1 mit der halben Einsatzmenge an Weißöl, Phosphorsäure und Lacton bei einer Sumpftemperatur von 170 °C aus. Die Zugabe des Lactons erfolgt konstant mit 30 ml/h ≈ 28 g/h. Die Produktabdestillation erfolgt mit der gleichen Rate wie in Beispiel 2, trotz halber Einsatzmenge.

```
Ausbeute nach Umsatz
von 5 mol Lacton (≙ 10 eq)  :  1 345,9 g (8,09 mol) ≙ 81 % Ausbeute
                               an 3-Methyl-2-pentylcyclopent-2-en-
                               1-on
typische Abtragungsraten
an Hastalloy-C4-Blechen     :  0,09 - 0,10 mm/Jahr
```

Beispiel 4

Man fährt den Versuch analog zu Beispiel 1, arbeitet aber mit Sumpftemperaturen von 150 °C. Das Lacton wird hierbei konstant mit 15 ml/h ≈ 14 g/h zugetropft.

```
Ausbeute nach Umsatz
von 10 mol Lacton         :  1 498,4 g (9,01 mol) ≙ 90 % Ausbeute
typische Abtragungsraten
an Hastalloy-C4-Blechen   :  0,01 - 0,02 mm/Jahr
```

Beispiel 5

Der Ansatz erfolgt analog zu Beispiel 4, verwendet aber nur ein 5 gew.-%iges Phosphorsäuregemisch in Weißöl bei gleicher Einsatzmenge an Phosphorsäure. In Angleichung zur verringerten Produktabdestillationsrate gibt man das Lacton gleichmäßig mit 12 ml/h ≈ 11 g/h zu.

```
Ausbeute nach Umsatz
von 10 mol Lacton         :  1 452,3 g (8,73 mol) ≙ 87 % Ausbeute
typische Abtragungsraten
an Hastalloy-C4-Blechen   :  um 0,01 mm/Jahr
```

Beispiel 6

Gemäß dem Beispiel 4 setzt man das Lacton bei Sumpftemperaturen von 150 °C um, verwendet jedoch 100 g (1 mol) 98 %iger Phosphorsäure in nur 400 g hochsiedendem Weißöl (Katalysatorgemisch 20 gew.-%ig an Phosphorsäure). Das Lacton kann hierbei mit der konstanten Rate von 20 ml/h ≈ 18,5 g/h zugetropft werden.

```
Ausbeute nach Umsatz
von 10 mol Lacton         :  1 489,9 g (8,96 mol) ≙ 90 % Ausbeute
typische Abtragungsraten
an Hastalloy-C4-Blechen   :  0,02 mm/Jahr
```

**Patentansprüche**

1. Verfahren zur Herstellung von 3-Methyl-2-pentyl-cyclopent-2-en-1-on durch katalytische Cyclodehydratisierung von 5-Hexyl-5-methyldihydro2(3H)-furanon (gamma-Methyl-gamma-decalacton) in Gegenwart von Phosphorsäure bei erhöhter Temperatur,
dadurch gekennzeichnet,
daß als Katalysator ein Gemisch von 5 bis 20 Gew.-% an Phosphorsäure und einem hochsiedenden Mineralöl eingesetzt, die Reaktion bei 145 bis 175 °C unter Entfernung des entstehenden Produktes sowie des Reaktionswassers unter Anlegen eines Unterdruckes durchgeführt wird.

2. Verfahren zur Herstellung von 3-Methyl-2-pentyl-cyclopent-2-en-1-on nach Anspruch 1,
dadurch gekennzeichnet,
daß die Reaktion vorzugsweise bei 150 bis 170 °C durchgeführt wird.

**Claims**

1. A process for the preparation of 3-methyl-2-pentyl-cyclopent-2-en-1-one by the catalytic cyclodehydration of 5-hexyl-5-methyldihydro-2(3H)-furanone (gamma-methyl-gamma-decalactone) in the presence of phosphoric acid at elevated temperature,
characterized in that
the catalyst used is a mixture of 5 to 20% by weight phosphoric acid and a high-boiling mineral oil, the reaction being performed with the application of a negative pressure at 145 to 175°C with the removal of the resulting product and the reaction water.

2. A process for the preparation of 3-methyl-2-pentyl-cyclopent-2-en-1-one according to claim 1,
characterized in that
the reaction is preferably performed at 150 to 170 C.

**Revendications**

1. Procédé d'obtention de la 3-méthyl-2-pentyl-cyclopent-2-en-1-one par cyclodishydratation catalytique de la 5-hexyl-5-méthyldihydro-2 (3H) furanone (gamma-méthyl-gamma-decalactone) en présence d'acide phosphorique à température élevée,
caractérisé en ce que
l'on met en oeuvre comme catalyseur un mélange de 5 à 20 % en poids d'acide phosphorique et d'huile minérale à haut point d'ébullition et que l'on effectue la réaction à 145 à 175° C en éliminant le produit qui s'est formé ainsi que l'eau de réaction tout en appliquant une dépression.

2. Procédé d'obtention de la 3-méthyl-2-pentyl-cyclopent-2-en-1-one selon la revendication 1,
caractérisé en ce que
la réaction est effectuée de préférence à 150 à 170°C.